# EUROPÄISCHE PATENTANMELDUNG

(11) **EP 0 842 642 A1**
(43) Veröffentlichungstag der Anmeldung: **20.05.1998**
(21) Anmeldenummer: 97100848.7
(22) Anmeldetag: 21.01.1997
(51) Int. Cl.: A61B 19/08

(54) **Doppel-Lochtuch für die Chirurgie an Extremitäten**

(30) Priorität: 18.11.1996 CH 2841/96
(71) Anmelder: Albrecht, Willi, 8730 Uznach (CH)
(72) Erfinder: Albrecht, Willi, 8730 Uznach (CH)

(57) **Zusammenfassung**

Das Abdecktuch dient zur sterilen Abdeckung einer Extremität während einer Operation, z.B. im Knie- oder Oberschenkelbereich. Es weist in der Längsachse zwei Ausschnitte mit elastischen Einsätzen auf, welche Öffnungen haben, deren Grösse im gedehnten Zustand etwa dem Durchmesser der Extremitäten entsprechen. Das Tuch wird so mehrfach zusammengelegt, dass die Öffnungen übereinander liegen. Dadurch kann es bei der Operation im zusammengelegten Zustand leicht über die Extremität geschoben und dann einfach ausgebreitet werden. Das gewährleistet eine kompakte Lagerung und eine einfache, sterile und schnelle Handhabung während der Operation.

## Beschreibung

Die Erfindung bezieht sich auf ein Abdecktuch zum sterilen Abdecken von Operationen an Extremitäten, z.B. im Oberschenkel-, Knie-, Unterschenkelbereich sowie im Bereich des oberen Sprunggelenkes.

Das Arbeiten mit den heutigen Abdeckungen ist aufgrund der lagerungstechnischen Gegebenheiten sehr zeitraubend, material- und wäscheaufwendig und liegt zum Teil an der Sterilitätsgrenze, und hat ausserdem weitere Nachteile:
- durch die längeren Abdeckungszeiten ergibt sich eine längere Narkosedauer, ein grösserer Medikamentenverbrauch, und eine grössere Belastung des Patienten;
- risikoreiche Abdeckung in Bezug auf die Sterilität;
- Verbrauch von zusätzlichem Einwegmaterial, d.h. mehr Abfall und grössere Umweltbelastung;
- durch den zusätzlichen Vebrauch von Stoffwäsche, welche auch wieder sortiert und gewaschen werden muss, steigt das Risiko, dass sich Leute an der mit Blut beschmutzten Wäsche an infektiösen Krankheiten anstecken könnten;
- durch den erhöhten Verbrauch an Wäsche ergibt sich ein Verbrauch von zum Teil sehr agressiven Waschmitteln (Blut, Eiter, Exkremente auswaschen) und erhöhte Belastung für die Umwelt durch Phosphate.

Es ist das Ziel der Erfindung, ein Abdecktuch zu schaffen, welches eine einfache Lagerung und bei der Operation eine schnelle, einfache und steril einwandfreie Abdeckung der Extremität eines Patienten ermöglicht, und bei welchem die weiteren oben genannten Nachteile entfallen.

Dieses Ziel wird erfindungsgemäss erreicht durch ein Abdecktuch gemäss den Patentansprüchen 1 bis 3 und durch eine Faltung und Vervendung des Abdecktuches gemäss den Patentansprüchen 5 bis 10.

Im folgenden wird ein Ausführungsbeispiel der Erfindung anhand von Zeichnungen beschrieben. Es zeigen:

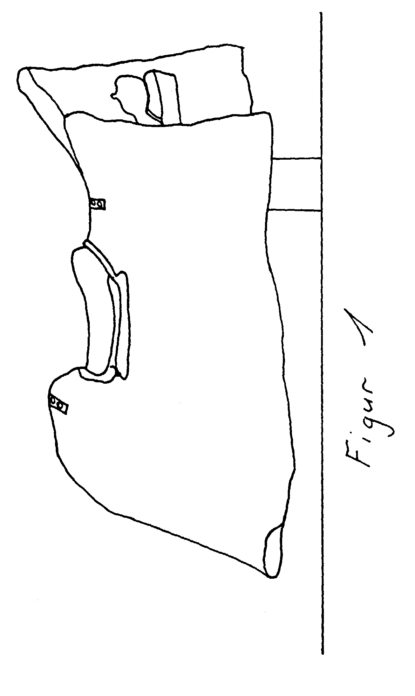

- Fig1.: das an einem Patienten auf einem Operationstisch applizierte erfindungsgemässe Abdecktuch;
- Fig.2: eine Gesamtansicht des Abdecktuches in ausgebreitetem Zustand;

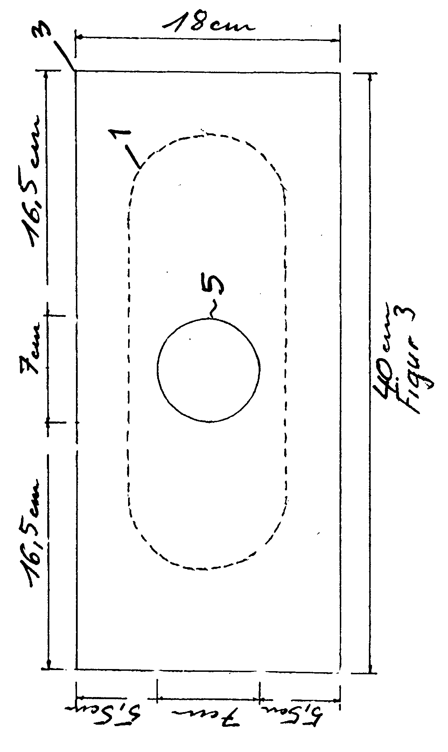

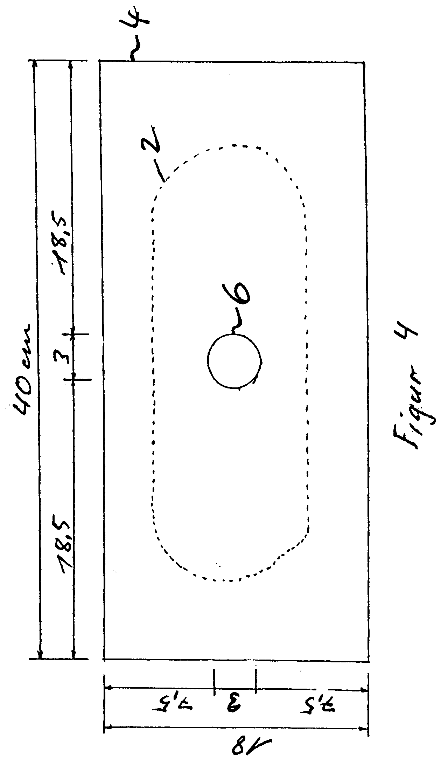

- Fig.3 und Fig.4: im Detail die elastischen Einsätze mit darunterliegenden Ausschnitten des Abdecktuches:

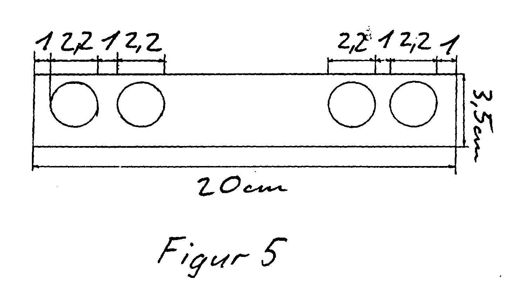

- Fig.5: im Detail einen der zusätzlichen Haltestreifen, durch welche Flüssigkeitsleitungen oder Kabel fixiert werden können;
- Fig.6a bis Fig.6f: die einzelnen Schritte bei der Zusammenlegung des Abdecktuches zur Lagerung und zu dessen Vorbereitung für die Operation.

### Detailbeschreibung

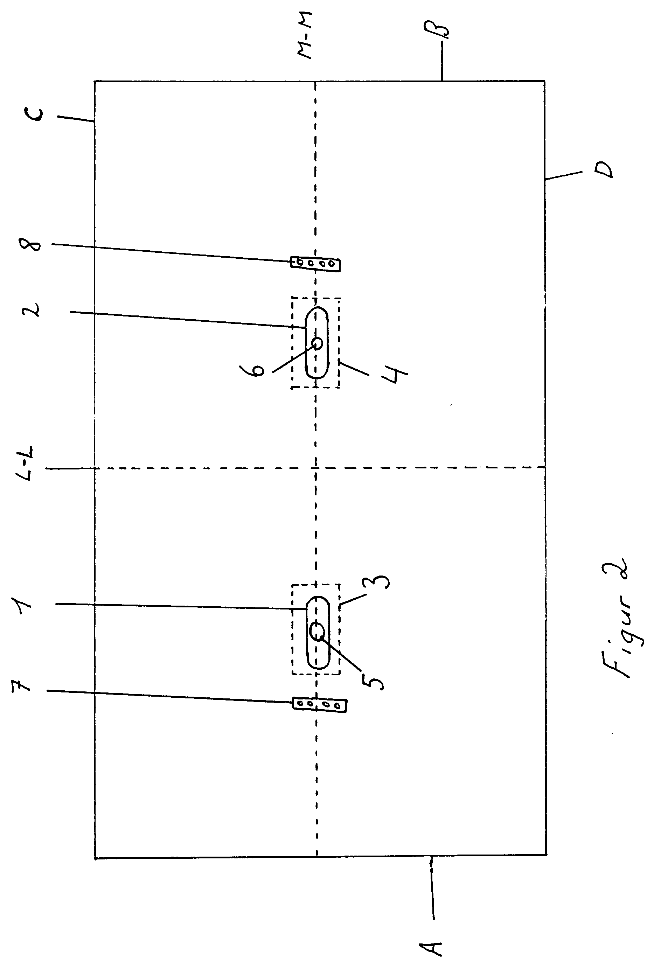

Das Abdecktuch, ein vertikales Extremitäten-Doppelloch-Tuch, besteht aus wasser- und zugfestem Material, hat etwa die doppelte Länge und die vierfache Breite eines Operationstisches (d.h. ca 4 m x 3 m). Wie in Fig.2 gezeigt, hat es zwei ellipsenförmige Ausschnitte (1, 2) beim oberen und unteren Drittel der Länge, welche von einer Seite her mit je einem Gummieinsatz (3, 4) überklebt sind. Die zwei Gummieinsätze, welche mit je einem Loch (5,6) verschiedener Grösse versehen sind, sind so auf den Ausschnitten (1, 2) angebracht, dass die Löcher in den Gummieinsätzen ungefähr in die Mitte der ellipsenförmigen Ausschnitte zu liegen kommen. Weiterhin vorgesehen sind Streifen (7, 8) aus ähnlichem Material, welche von der Rückseite her an dem Tuch befestigt werden und sich zwischen je einer Tuchkante (A, B) und einem der Gummieinsätze (3, 4) sowie in der Mitte des Tuches befinden. Sie können die Masse z.B. von 20 cm x 3,5 cm haben und verfügen über etwa 3 bis 4 kreirunde Löcher, welche einen Minimaldurchmesser von 2 cm haben. Diese Löcher dienen zur Fixierung von Ableitungen oder elektrischen Kabeln während der Operation.

### Applikation:

Die Handhabung ist auch für eine unroutinierte OP-Schwester leicht auszuführen. Sie muss nur durch die beiden Gummimanschetten (3, 4) greifen, den Vorfuss des Patienten umfassen und beide Manschetten über das Fussgelenk ziehen, danach die oberschenkelnahe Manschette bis über das Knie nach oben schieben, Tuchrand A nach kopfwärts, Tuchrand B fusswärts fallen lassen. Damit ist die Abdeckung schon fertig.

### Zusammenlegen des Abdecktuches:

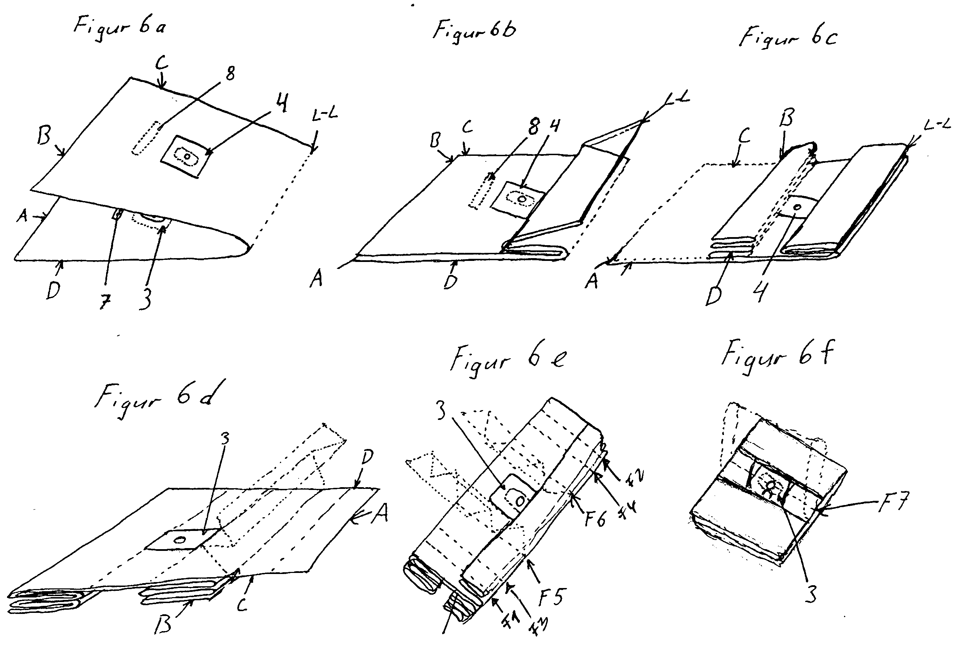

Um den Umgang mit einem so grossen Tuch alleine bewältigen zu können und um eine kompakte und sterile Lagerung zu ermöglichen sowie um die schnelle und einfache Handhabung zu erreichen, wird jedes Tuch so zusammengelegt, wie es im folgenden anhand der Figuren 6a bis 6f beschrieben ist.

Zunächst wird das Tuch, wie es ausgebreitet in Fig.2 gezeigt ist, längs der gestrichelt gezeigten Linie L-L einmal zusammengefaltet, so dass die beiden Gummieinsätze 3 und 4 übereinander zu liegen kommen, wie in Fig.6a gezeigt. Es liegt dann auch die Kante A bei der Kante B, und die beiden Streifen 7 und 8 liegen auch übereinander. Wesentlich ist aber, dass die Öffnungen 5 und 6 der beiden Gummieinsätze 3 und 4 übereinander liegen, so dass sie eine gemeinsame Öffnung bilden und der Gummieinsatz 4 mit der kleineren Öffnung 6 oben liegt.

Danach wird die Hälfte des zunächst einfach übereinanderliegenden Abdecktuches, an welcher sich nun aussen die Faltkante L-L befindet, harmonikaförmig zusammengelegt, wie in Fig. 6b gezeigt. Die Gummieinsätze 3 und 4 müssen dabei aber frei bleiben.

Im nächsten Schritt wird das noch flach liegende Viertel des Tuches, welches zwischen der Kante B und dem Einsatz 4 liegt, ebenfalls harmonikaförmig zusammengelegt gemäss Fig.6c, wobei auch wieder der Gummieinsatz 4 frei bleiben muss.

Dann wird das teilweise zusammengelegte Tuch so gewendet, dass die bisherige Oberseite unten zu liegen kommt und das Viertel des Tuches zwischen der Kante A und dem Einsatz 3, welches noch flach ist, nach oben zu liegen kommt. Dieser noch flache Teil wird nun auch harmonikaförmig gefaltet, wie in Fig.6d gezeigt.

Nunmehr hat das Abdecktuch die Form eines langen Streifens mit harmonikaförmigen Falten an den Längsenden und freiliegenden Einsätzen 3 und 4 in der Mitte, siehe Fig.6e. Zum Schluss werden nochmals zwei harmonikaförmige Faltungen gemacht längs den gestrichelt gezeichneten Linien F1 bis F6, wobei diesmal die Faltungen quer zu den bisherigen Faltungen erfolgen.

Wiederum müssen in der Mitte die Einsätze 3 und 4 mit ihren übereinanderliegenden Öffnungen freibleiben. Am Schluss hat das mehrfach gefaltete Abdecktuch eine rechteckige Form von etwa 60 cm x 40 cm. Da dies der Handlichkeit wegen immer noch zu gross ist, wird das Abdecktuch noch einmal entlang der gestrichelt gezeichneten Linie F7 gefaltet.

In dieser zusammengelegten Form wird das Tuch in einer Klarsichthülle steril verpackt und gelagert. Zum Gebrauch im Operationssaal wird dann nur die Plastikhülle aufgerissen, die OP-Schwester nimmt das zusammengefaltete Tuch heraus, greift mit der Hand durch die Gummieinsätze 3 und 4, umfasst den Vorfuss des Patienten und stülpt das Tuch darüber, zieht das Tuch auseinander und schiebt den Gummieinsatz mit der grösseren Öffnung über das Knie bis zum oberen Drittel des Oberschenkels.

Somit ist eine sterile und sehr effiziente Abdeckung der Extremität eines Patienten gewährleistet.

Für die Abmessungen des Abdecktuches sind die zweifache Länge und vierfache Breite eines Operationstisches deshalb vorgesehen, weil dadurch eine Arbeit mit einem Röntgenapparat möglich wird, ohne dass jedesmal zusätzliche sterile Wäsche verwendet werden muss.

Alternative Ausgestaltung des Abdecktuches:

Für bestimmte Anwendungen, z.B. dynamische Hüftschraube, Gamma-Nagel, Oberschenkel-Marknagel, Oberschenkelfraktur usw. kann das Abdecktuch folgendermassen ausgestaltet werden: Während der eine Gummieinsatz 3 mit einem kreisrunden Loch 5 versehen wird, bleibt der andere Gummieinsatz 4 zunächst ohne Loch (also KEINE Öffnung 6). Das Tuch wird dann auch zusammengelegt, wie in Fig.6a bis 6f gezeigt, so dass also die beiden Gummieinsätze 3 und 4 übereinander liegen und die Öffnung 5 des Gummieinsatzes 3 freiliegt.

Vor der Anwendung des Abdecktuches kann nun der Arzt mit einer Schere nach Bedarf, durch die Öffnung 5 im Gummieinsatz 3 hindurch, ein Loch von der erforderlichen Grösse in den Gummieinsatz 4 hineinschneiden (evtl. auch einfach ein länglicher oder kreuzförmiger Schnitt). Danach kann das Abdecktuch von der OP-Schwester appliziert werden wie oben beschrieben.

## Patentansprüche

1. Abdecktuch aus reissfestem und wasserdichten Material zur Abdeckung einer Extremität bei Operationen, gekennzeichnet durch folgende Merkmale:
- Abmessungen von mindestens der Länge und Breite einer Operationsfläche;
- zwei ellipsenförmige oder kreisrunde Ausschnitte (1, 2) auf einer mittleren Längsachse (M-M) des Abdecktuches; und
- zwei Einsätze (3, 4) aus elastischem Material, welche über den Ausschnitten (1, 2) befestigt sind, wobei mindestens einer der Einsätze eine runde Öffnung (5, 6) hat, welche etwa in der Mitte des betreffenden Ausschnittes liegt.

2. Abdecktuch gemäss Patentanspruch 1, dadurch gekennzeichnet, dass die beiden Einsätze (3, 4) aus elastischem Material unterschiedlich grosse, etwa kreisförmige Öffnungen (5, 6) haben, welche in gedehntem Zustand je dem Querschnitt einer Extremität an ihrem oberen und unteren Ende entsprechen.

3. Abdecktuch gemäss Patentanspruch 1, dadurch gekennzeichnet, dass nur einer der beiden Einsätze (3) aus elastischem Material eine etwa kreisförmige Öffnung (5) hat, während der andere Einsatz (4) keine Öffnung aufweist, so dass bei diesem anderen Einsatz (4) je nach Bedarf eine Öffnung mit einem Schneidwerkzeug eingeschnitten werden kann.

4. Abdecktuch gemäss Patentanspruch 1, dadurch gekennzeichnet, dass seine Länge etwa der doppelten Länge eines Operationstisches entspricht, dass seine Breite etwa der vierfachen Breite eines Operationstisches entspricht, und dass die beiden Ausschnitte (1, 2) und die beiden Einsätze (3, 4) aus elastischem Material auf einer mittleren Längsachse (M-M) bei etwa je einem Drittel und zwei Dritteln der Länge liegen.

5. Abdecktuch gemäss Patentanspruch 1, gekennzeichnet durch mindestens einen zusätzlichen Streifen (7, 8) aus reissfestem Material, welcher im oberen oder unteren Drittel des Abdecktuches befestigt ist und mehrere Öffnungen zur Durchführung und Halterung von Versorgungsleitungen oder Kabeln aufweist.

6. Abdecktuch gemäss Patentanspruch 2, dadurch gekennzeichnet, dass das Abdecktuch zur Vorbereitung und Lagerung folgendermassen dreifach zusammengelegt ist:
a) erstens: einmalige Faltung des Tuches längs einer Mittellinie (L-L) quer zur Längsachse (Fig.6a);
b) zweitens: harmonikaförmige Zusammenlegung von beiden Seiten her (L-L; A, B), parallel zur ersten Faltung (Fig.6b, 6c, 6d); und
c) drittens: harmonikaförmige Zusammenlegung von beiden Enden her (F1...F6), aber quer zur Faltrichtung gemäss a) und b) (Fig.6e);
wobei im zusammengefalteten Abdecktuch die beiden Einsätze (3, 4) übereinander liegen und ihre Öffnungen (5, 6) eine gemeinsame, freiliegende Öffnung bilden.

7. Abdecktuch gemäss Patentanspruch 6, dadurch gekennzeichnet, dass die harmonikaförmige Zusammenlegung gemäss b) folgende Aufteilung hat:
- Faltung der einen Hälfte des Abdecktuches, welches die geschlossene erste Faltkante (L-L) enthält, sowie Faltung eines Viertels des Abdecktuches, welches zwischen einer der Endkanten (B) und einem der beiden elastischen Einsätze (4) liegt, in Harmonikaform auf der Seite des einen elastischen Einsatzes (4) (Fig.6d, 6c); und
- Faltung des letzten Viertels des Abdecktuches, welches zwischen der anderen Endkante (A) und dem anderen der beiden elastischen Einsätze (3) liegt, in Harmonikaform auf der Seite des anderen elastischen Einsatzes (3) (Fig.6d);
so dass die zwei harmonikaförmigen Faltungen mit je einer Endkante (A, B), auf entgegengesetzten Seiten des Abdecktuches liegen.

8. Abdecktuch gemäss Patentanspruch 3, dadurch gekennzeichnet, dass das Abdecktuch zur Vorbereitung und Lagerung folgendermassen dreifach zusammengelegt ist:
a) erstens: einmalige Faltung des Tuches längs einer Mittellinie (L-L) quer zur Längsachse (Fig. 6a);
b) zweitens: harmonikaförmige Zusammenlegung von beiden Seiten her (L-L; A, B), parallel zur ersten Faltung (Fig.6b, 6c, 6d); und
c) drittens: harmonikaförmige Zusammenlegung von beiden Enden her (F1...F6), aber quer zur Faltrichtung gemäss a) und b) (Fig.6e);
wobei im zusammengefalteten Abdecktuch die beiden Einsätze (3, 4) übereinander liegen, so dass durch die eine Öffnung (5) in dem einen Einsatz (3) eine Öffnung in den anderen Einsatz (4) hineingeschnitten werden kann.

9. Verwendung des Abdecktuches gemäss Patentanspruch 2, dadurch gekennzeichnet, dass das Abdecktuch zur Vorbereitung und Lagerung folgendermassen zusammengelegt wird:
a) einmaliges Falten des Tuches längs einer Mittellinie (L-L) quer zur Längsachse, so dass die beiden elastischen Einsätze (3, 4) übereinander liegen und ihre Öffnungen (5, 6) zur Deckung kommen, wobei sie eine gemeinsame Öffnung bilden (Fig.6a);
b) harmonikaförmiges Zusammenlegen des bisher einmal gefalteten Tuches von den beiden Seiten her (L-L; A, B), so dass die gemeinsame Öffnung der beiden elastischen Einsätze (3, 4) weiterhin frei bleibt (Fig. 6b, 6c, 6d); und
c) nochmaliges harmonikaförmiges Zusammenlegen des teilgefalteten Abdecktuches von beiden Enden her, aber quer zur bisherigen Faltrichtung, wobei wieder die gemeinsame Öffnung der beiden elastischen Einsätze (3, 4) freibleibt (Fig.6e).

10. Verwendung gemäss Patentanspruch 9, dadurch gekennzeichnet, dass im Schritt b) das harmonikaförmige Zusammenlegen folgendermassen erfolgt:
- Falten der einen Hälfte des Abdecktuches, welches die geschlossene erste Faltkante (L-L) enthält, in Harmonikaform (Fig.6b);
- Falten des einen noch flachen Viertels des Tuches, welches zwischen einer der Endkanten (B) und einem der beiden elastischen Einsätze (4) liegt, in Harmonikaform (Fig.6c);
- Wenden des teilgefalteten Abdecktuches von der Oberseite auf die Unterseite; und
- Falten des noch flachen letzten Viertels des Abdecktuches, welches zwischen der anderen Endkante (A) und dem anderen der beiden elastischen Einsätze (3) liegt, in Harmonikaform (Fig.6d);
so dass die zwei harmonikaförmigen Faltungen mit je einer Endkante (A, B), auf entgegengesetzten Seiten des Abdecktuches liegen, und die übereinanderliegenden Öffnungen (5, 6) der beiden elastischen Einsätze (3, 4) frei bleiben.

11. Verwendung des Abdecktuches gemäss Patentanspruch 3, dadurch gekennzeichnet, dass das Abdecktuch zur Vorbereitung und Lagerung folgendermassen zusammengelegt wird:
a) einmaliges Falten des Tuches längs einer Mittellinie (L-L) quer zur Längsachse, so dass die beiden elastischen Einsätze (3, 4) übereinander liegen (Fig.6.a);
b) harmonikaförmiges Zusammenlegen des bisher einmal gefalteten Tuches von den beiden Seiten her (L-L; A, B), so dass die beiden Einsätze (3, 4) aus elastischem Material frei bleiben (Fig.6b, 6c, 6d); und
c) nochmaliges harmonikaförmiges Zusammenlegen des teilgefalteten Abdecktuches von beiden Enden her, aber quer zur bisherigen Faltrichtung, wobei wieder die beiden Einsätze (3, 4) aus elastischem Material frei bleiben (Fig.6e);
so dass im zusammengelegten Abdecktuch durch die eine Öffnung (5) in dem einen Einsatz (3) eine Öffnung in den anderen Einsatz (4) hineingeschnitten werden kann.
